# EUROPEAN PATENT SPECIFICATION

(11) **EP 0 459 954 B1**
(45) Date of publication and mention of the grant of the patent: **27.03.1996**
(21) Application number: 91830188.8
(22) Date of filing: 09.05.1991
(51) Int. Cl.: A61M 25/06

(54) **One-way intravenous catheter with needle guard**
Einwegvenenkatheter mit Nadelschutzhulse
Cathéter intraveineux à usage unique avec protecteur pour l'aiguille

(30) Priority: 29.05.1990 IT 3579290 U
(43) Date of publication of application: 04.12.1991
(73) Proprietor: ETHICON S.P.A., I-00040 Pratica di Mare (Roma) (IT)
(72) Inventor: Rossetti, Alessandro, I-00040 Pratica di Mare (Roma) (IT)
(74) Representative: Banchetti, Marina

(56) References cited:
- EP-A- 0 281 421
- EP-A- 0 343 803
- US-A- 3 595 230
- US-A- 4 917 669

## Description

The present invention relates to a one-way intravenous catheter with needle guard. More specifically, the invention relates to a one-way catheter assembly which allows the retraction of the needle within a protective guard as soon as the catheter tube is placed into a blood vessel, thus protecting the user from accidental needle injury.

The conventional one-way intravenous catheter originates from the need of infusing pharmaceutical liquids (flebochlysis) or blood (transfusion) into the venous system, and of drawing blood therefrom (donation).

An intravenous catheter assembly typically comprises a thin teflon tube (cannula), of a size which depends on the required use, attached to a plastic hub, and provided with standard connecting means for connection to the infusion or blood drawing sets. The catheter tube is inserted into the vein of a patient by means of an inner coaxial stainless steel needle, which is withdrawn by retracting it from the catheter tube right after the insertion of the catheter into the patient's vein. The needle is attached to a separate plastic support.

One-way catheters are generally employed in hospitals for administering pharmaceutical solutions directly into the venous system. The only way of the catheter is connected to an infusion set, which is in its turn connected to the container of the liquid to be infused. After that the catheter has been safely placed into the patient's vessel, the needle is withdrawn from the catheter and disposed of. In order to provide the necessary sterility to the catheter and needle assembly, and to allow a safe handling of the device before use, the latter is normally packaged in sterile envelopes or sheaths.

US-A-3 595 230 which is considered to represent the closest prior art with regard to the present invention discloses a one-way intravenous catheter comprising a catheter tube or cannula and a needle positioned therein, attached to a needle housing having a peripheral gripping means in the form of a thumb tab vertically projecting from said needle housing. The assembly of needle housing and catheter tube is protected by a tubular sheath or needle guard almost completely enclosing said assembly, which needle guard is provided with a longitudinal slot for guiding the sliding movement of said assembly with respect to the needle guard. Both the needle and its housing are provided with longitudinal elements cooperating with said longitudinal slot in guiding the sliding movement.

By means of the said relative sliding movement, the catheter and needle assembly may be displaced from an initial position, wherein the assembly is completely protected within the needle guard, to a use position, wherein catheter and needle are exposed for carrying out the venipuncture. During said operation, the needle housing is pushed forward by means of the thumb tab mentioned above.

In order to correctly handle a catheter device, after the venipuncture the user should dispose of the needle, but very often he connects the infusion set as a first step, and he tapes the catheter on the patient's skin, and just drops the needle nearby, so that he can retrieve it and dispose of it later.

The various handlings of the needle, once it has been removed, bring about considerable risk of accidental needle injury, which may have serious consequences if the needle is infected by the patient's blood. This problem is particularly critical in the hospital departments where serious infectious diseases such as hepatitis or AIDS are treated.

In order to reduce the danger coming from used needles, there have been provided plastic sheaths or caps that should be employed to cover the needle right after use, but this operation may easily cause accidental injury right when the sheath is being placed again on the needle.

A device directed to the solution of the above-mentioned problem is disclosed in EP-A-0 392 765, whose priority date is April 10, 1989. Such EP-A is comprised in the state of the art within the meaning of Article 54 (3) and (4) EPC with respect to the present application.

The one-way catheter disclosed in the above-mentioned patent application comprises a catheter tube or cannula with the relevant hub, a needle located within said cannula and attached to a specially designed plastic housing, a protective needle guard provided with a longitudinal slot which may slide with respect to said housing so as to completely cover the needle, and a removable protective sheath which surrounds the needle and catheter tube prior to use. As initially assembled, the needle guard is completely retracted with respect to the needle housing, and the needle and catheter tube are covered by the above mentioned sheath, which is releasably connected by means of a flange to said needle guard. Prior to use, the sheath is removed and the catheter is inserted into the patient's vessel, as usual. Once the tip of the needle has reached the blood vessel, the user, while holding the device by the needle housing, advances the above mentioned tubular needle guard, thereby disengaging the catheter hub from the needle and causing the retraction of the needle within the needle guard starting from the rear end of the needle.

Thus, the immediate protection of the needle right after use is obtained without requiring the user's hands to pass close to the needle tip, and therefore allowing a quite safe operation. The disclosed device, while affording the required safety, has, however, a rather complex structure and is, accordingly, quite expensive. In particular two additional elements are required, i.e. the protective sheath and the tubular needle guard, whose construction is particularly complicate.

The object of the present invention is therefore to provide a one-way intravenous catheter which allows the same safety and ease of operation as the catheter of the prior art, while being of a simpler construction and involving lower production costs. It is evident that, in order to result in a real contribution to the safety against blood borne diseases, a device of the kind disclosed must be reasonably cheap, so as to be available to a wide market.

According to the present invention, there is proposed to supply a conventional one-way catheter with a housing element for the needle, substantially in the form of a hollow cylinder, with a peripheral finger grip coaxial to said cylinder and connected to it by means of a longitudinal connecting element, and with one simple tubular needle guard longitudinally cut by a guiding slot, slidable on said longitudinal connecting element of the needle housing, so as to completely surround all the elements of the catheter but the above-mentioned peripheral finger grip. The shape of the said longitudinal connecting element and that of the needle guard slot which cooperates with the former are such as to have an irreversibly locked position with the needle completely retracted within the needle guard.

Since the needle guard has a tubular shape, open at least at the front end, it may slide with respect to the needle housing, and therefore with respect to the whole catheter, between two extreme positions, the first one of complete retraction, with the needle and catheter tube fully uncovered (use position) and the other of complete extension, with the needle (and possibly the catheter tube) completely covered by the needle guard.

The catheter according to the invention does not require the use of a further element such as a sheath, to be placed on the needle in the initial assembly, as the needle is already protected by the tubular needle guard in its fully extended position. Prior to use, the needle guard is pushed backwards, thus uncovering the needle and the catheter tube, and after that the catheter has been placed into the blood vessel, the needle guard is returned to its extended position, thus protecting the needle. At the end of the stroke the locking mechanism of the needle guard locks the needle in the needle housing thereby avoiding any accidental needle injury.

Accordingly, the present invention specifically provides a one-way intravenous catheter with needle guard comprising: -) a catheter tube or cannula attached to a catheter hub tapered on both its internal and external surfaces; -) a needle whose outer diameter is sized to fit in said catheter tube, attached to a needle housing substantially in the form of a hollow cylinder, with peripheral finger gripping means connected to the central body of said needle housing by means of a longitudinal connecting element; -) a tubular needle guard whose length is greater than the length of the needle and whose inner diameter is sufficient for it to surround all the elements of the catheter but said peripheral finger gripping means, said needle guard being provided with a longitudinal guiding slot cooperating with said longitudinal connecting element for sliding relative to said needle housing, wherein locking means are provided on said slot and connecting element for locking the needle guard in its position totally advanced with respect to the catheter, and wherein the front portion of said needle housing is sized to engage the tapered internal surface of said catheter hub, characterised in that said finger gripping means is in the form of an outer coaxial element, connected to the central body of said needle housing by means of said longitudinal connecting element.

Preferably, the central cylindrical portion of said needle housing is provided with two transversal guiding ribs whose outer diameter is sized to fit the internal surface of the tubular needle guard. As it will be clear with reference to the enclosed drawings, the function of said two transversal ribs is to smoothly guide the sliding of the needle within the needle guard while keeping it perfectly coaxial with the latter. In an alternative embodiment of the invention, there are provided four longitudinal guiding ribs, projecting from said central cylindrical portion of the needle housing and uniformly spaced around it, the height of said ribs being such that said central cylindrical portion fits the internal surface of the tubular needle guard.

According to a preferred embodiment of the invention, said peripheral finger gripping means has a partially interrupted cylindrical internal surface, and a substantially parallelepipedal outer surface with two contoured opposite faces.

The longitudinal connecting element which connects the central body of said needle housing to the peripheral finger gripping means, and which cooperates with said longitudinal slot for guiding said needle guard, is preferably made of two subsequent separate portions both tapered to a greater width towards the central portion of said connecting element, and separated by an aperture in said central portion. In this embodiment the locking means provided on said connecting element are represented by said aperture in the central portion of said connecting element, while the corresponding locking means on the needle guard are represented by two opposed teeth projecting along the guiding slot. Said teeth are such as to irreversibly engage with said aperture in the central portion of the connecting element.

Preferably, said longitudinal slot is provided with a second couple of opposed teeth having a rounded profile and located towards the front end of said needle guard with respect to said two first opposed teeth. As it will be clear with reference to the enclosed drawings, said second couple of teeth provides a non-irreversible pre-lock on which the catheter assembly is positioned before use, in order to prevent accidental sliding of the needle and catheter tube out of the needle guard before use.

In a specific embodiment of the invention, the catheter assembly also comprises a tab projecting upwards from the front end of said tubular needle guard, which may be held by the user when retracting or advancing the needle guard with respect to the needle housing.

The central cylindrical portion of said needle housing is preferably made of a transparent or translucent material, so that said cylindrical central portion may be used as a flash chamber allowing the flow of blood to be apparent as soon as the needle reaches a blood vessel. The flash chamber may be closed by a microporous plug.

The structure of the catheter assembly according to the invention, as well as the relevant use and advantages, will be made clear with reference to some preferred embodiments thereof, which are shown in the enclosed drawings, wherein:
Figure 1 is a front view of a first embodiment of a one-way catheter assembly with needle guard according to the invention;
Figure 2 is a front view of the needle guard of the catheter assembly of Figure 1;
Figure 3 is a front view of the needle housing of the catheter assembly of Figure 1;
Figure 4 is a view of the same catheter assembly of Figure 1 taken from the opposite side and without the catheter tube;
Figure 5 is a front view of a second embodiment of the needle guard of the catheter assembly according to the invention;
Figure 6 is a lateral view of the needle guard of Figure 5 taken from the right-hand side and rotated by 90°;
Figure 7 is a front view of the central portion of a second embodiment of the needle housing according to the invention; and
Figure 8 is a cross sectional view of the needle housing of Figure 7, taken along the line A-A.

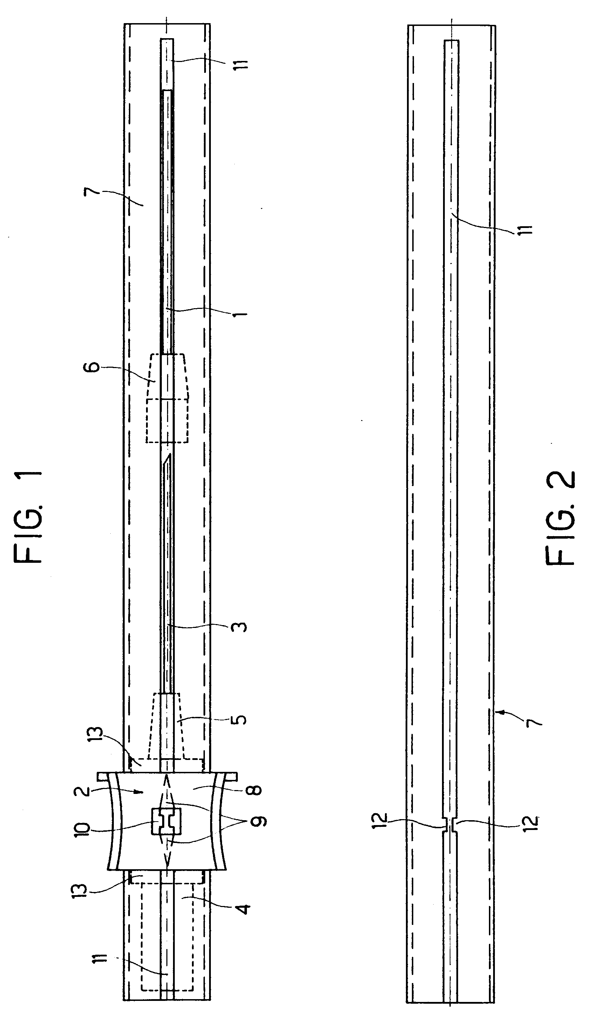

Figure 1 shows a catheter according to the invention with most of its elements and with the catheter tube 1 shown separate for a better understanding.

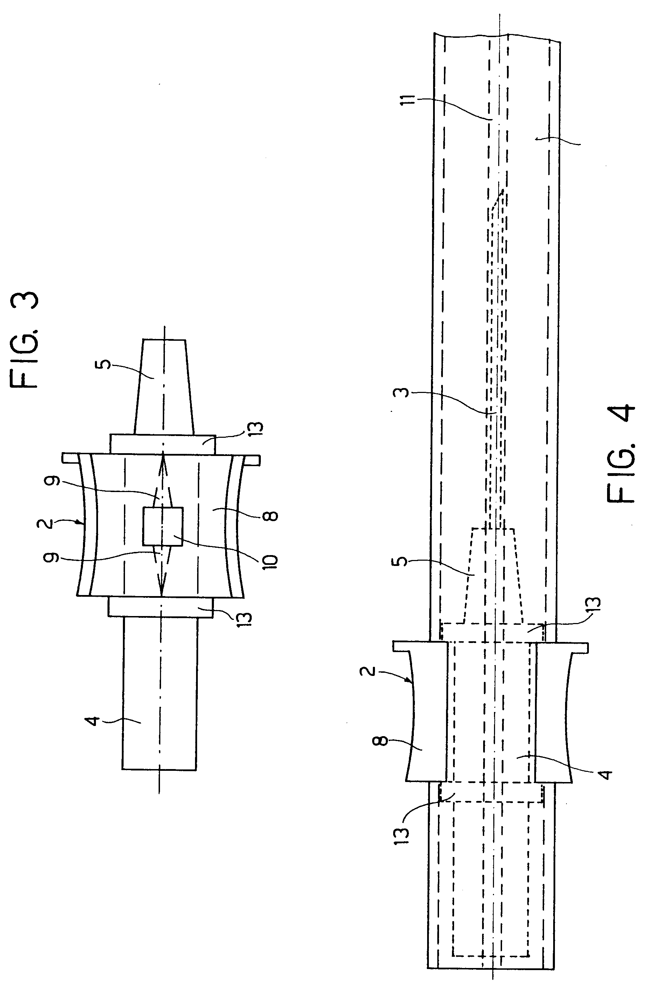

The housing 2 of the needle 3 (shown in detail in Figure 3) comprises a central body 4 substantially cylindrical, which is attached to the needle 3. The front portion 5 of the needle housing 2 is slightly tapered (male luer ISO) so as to perfectly engage the tapered internal surface of the catheter hub 6 (female luer ISO).

The central body 4 of the needle housing 2 is made of a transparent or translucent material, so as to be used as a flash chamber, and is generally closed by a microporous plug (not shown). Said plug allows the passage of gases while preventing the liquid flow out of the flash chamber and is made of, e.g., non-toxic porous polyethylene. The central body 4 may coaxially slide within a needle guard 7, made of transparent plastic material.

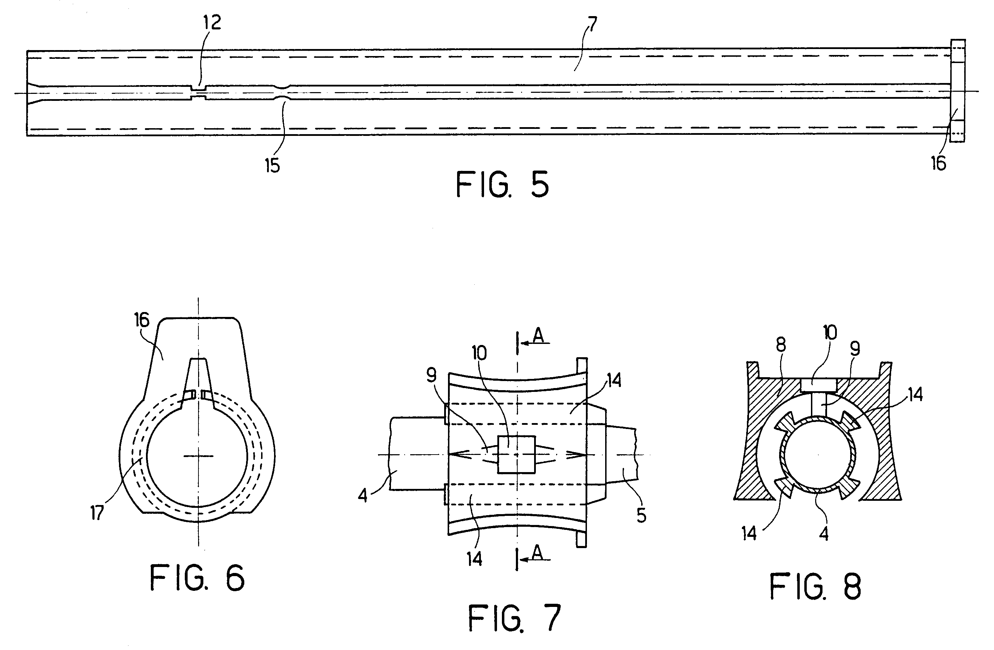

The central body 4 is surrounded by a peripheral finger gripping element 8 coaxial with the catheter, which has an inner interrupted cylindrical surface sized to fit around the tubular needle guard, and an outer surface substantially parallelepipedal, with two contoured opposite sides, so as to be easily gripped by the user with the index and the thumb. The gripping element 8 is the only element of the catheter assembly which is not contained within the needle guard 7, but extends around it and almost completely surrounds it. In particular, the gripping element 8 is seen as an integral element in the front views of figures 1 and 3, while in the opposite view of figure 4 only two separate sections thereof are visible.

The finger gripping element 8 is connected to the central body 4 of the needle housing 2 by means of a longitudinal connecting element divided into two separate sections 9 shown by broken lines in figures 1 and 3. The cross-section of both sections 9 is tapered towards the central portion of the connecting element, from a smaller to a greater width, and the central portion is interrupted by a square aperture 10.

The needle guard 7, shown in detail in figure 2, is supplied with a longitudinal guiding slot 11, which engages with the two sections 9 of the longitudinal connecting element. The slot 11 has two opposed teeth 12 which are part of an irreversible locking mechanism, together with the aperture 10 on the needle housing 2. Figure 1 shows the needle housing 2 in the locked position, with the teeth 12 engaged in the aperture 10. The tapered cross-section of the sections 9 of the connecting element is designed to guide the two teeth 12 apart from each other while the needle housing 2 slides with respect to the needle guard 7, until the locking position is reached.

When advancing the needle 3 and catheter 6 and 1 prior to use, the longitudinal slot 11 cooperates with the two sections 9 of the connecting element in guiding the needle housing 2 out of the needle guard 7. After that the catheter tube 1 has been placed into the patient's vein the same guiding system helps the retraction of the needle 3 within the needle guard 7. When the needle housing 2 is pushed towards a position fully retracted with respect to the needle guard 7, the two teeth 12 on the slot 11 cooperate with the sections 9 of the connecting element and irreversibly lock the catheter assembly in the position shown in figure 1. The latter position is reached after use, when the catheter assembly, deprived of the catheter tube 1 and hub 6, is going to be disposed of.

In order to obtain a perfect coaxiality of the needle housing 2 with the needle guard 7, two transversal guiding ribs 13 are provided around the central body 4 of the needle housing 2. The outer diameter of the guiding ribs 13 is such that they perfectly fit within the tubular needle guard 7.

A different embodiment of the needle housing 2 is shown in figures 7 and 8, where corresponding elements are shown by the same numerals as in the previous figures. The four longitudinal guiding ribs 14 have the same function as the two transversal guiding ribs 12 of figures 1, 3 and 4.

A second embodiment of the needle guard of the invention is shown in figures 5 and 6, where corresponding elements are shown by the same numerals as in the previous figures. In this embodiment, a pre-lock formed by the teeth 15 has been added, in order to provide a stable position in which the catheter assembly can be stored before use, thereby avoiding that the needle 3 accidentally slides out of the housing 7 e.g. during transport. Since the pre-lock position must not be irreversible, the two teeth 15 have a rounded profile.

A further feature of the embodiment shown in figures 5 and 6 is the tab 16, projecting upwards from the front end of the needle guard 7. The tab 16 may be gripped while making the needle guard 7 slide with respect to the needle housing 2. As shown in figure 6, the inner diameter of the collar 17 of the tab 16 is slightly smaller than inner diameter of the needle guard 7. When the catheter hub 6, mounted on the needle 3, is pushed forward for use, it is made to pass the narrowed section of the collar 17 by means of a slight pressure. As a result, the catheter hub 6 and tube 1 are prevented from being retracted together with the needle 3 after that the catheter tube has reached the blood vessel, as the catheter hub 6 abuts on the collar 17.

It is evident from the foregoing that the catheter assembly according to the invention affords the highest safety against accidental needle injury, as it prevents the needle tip from coming into contact with the user's hands either before use or after. In particular, no additional sheath is to be removed before use, and the used needle is retracted within the needle guard as it is extracted from the patient's blood vessel.

Furthermore, the construction of the catheter assembly according to the invention is particularly simple, and the relevant production costs may be expected to be reasonably low.

## Claims

1. One-way intravenous catheter with needle guard comprising:
- a catheter tube or cannula (1) attached to a catheter hub (6) tapered on both its internal and external surfaces;
- a needle (3) whose outer diameter is sized to fit in said catheter tube (1), attached to a needle housing (2) substantially in the form of a hollow cylinder, with peripheral finger gripping means (8) connected to the central body (4) of said needle housing (2) by means of a longitudinal connecting element (9);
- a tubular needle guard (7) whose length is greater than the length of the needle (3) and whose inner diameter is sufficient for it to surround all the elements of the catheter but said peripheral finger gripping means (8), said needle guard (7) being provided with a longitudinal guiding slot (11) cooperating with said longitudinal connecting element (9) for sliding relative to said needle housing (2), wherein locking means (12, 10) are provided on said slot (11) and connecting element (9) for locking the needle guard (7) in its position totally advanced with respect to said needle housing (2), and wherein the front portion (5) of said needle housing (2) is sized to engage the tapered internal surface of said catheter hub (6), characterised in that said finger gripping means (8) is in the form of an outer coaxial element, connected to the central body (4) of said needle housing (2) by means of said longitudinal connecting element (9).

2. Catheter according to claim 1, wherein the central cylindrical portion (4) of said needle housing (2) is provided with two transversal guiding ribs (13) whose outer diameter is sized to fit the internal surface of said tubular needle guard (7).

3. Catheter according to claim 1, wherein the cylindrical portion (4) of said needle housing (2) is provided with four longitudinal guiding ribs (14), the height of said ribs being such that said central cylindrical portion (4) fits the internal surface of the tubular needle guard (7).

4. Catheter according to any one of claims -1-3, wherein said peripheral finger gripping means (8) has a partially interrupted cylindrical internal surface, and a substantially parallelepipedal outer surface with two contoured opposite faces.

5. Catheter according to any one of claims 1-4, wherein said longitudinal connecting element (9) which connects the central body (4) of said needle housing (2) to the peripheral finger gripping means (8), and which cooperates with said longitudinal slot (11) for guiding said needle guard (7), is made of two subsequent separate portions both tapered to a greater width towards the central portion of said connecting element (9), and separated by an aperture (10) in said central portion, and wherein said locking means provided on said connecting element consist of said aperture (10) in the central portion of said connecting element (9), while the corresponding locking means on said needle guard consist of two opposed teeth (12) projecting along said guiding slot (11).

6. Catheter according to any one of claims 1-5, wherein said longitudinal slot (11) is provided with a second couple of opposed teeth (15) having a rounded profile and located towards the front end of said needle guard (7) with respect to said two first opposed teeth (12).

7. Catheter according to any one of claims 1-6, also comprising a tab (16) projecting upwards from the front end of said tubular needle guard (7).

8. Catheter according to any one of claims 1-7, wherein said central cylindrical portion (4) of said needle housing (2) is made of a transparent or translucent material.

9. Catheter according to any one of claims 1-8, also comprising a microporous plug closing the rear end of said central cylindrical portion (4).

## Patentansprüche

1. Ein intravenouser Einweg-Katheter mit einer Nadelschutzhülse, enthaltend:
- ein an eine an derer inneren und äusseren Fläche verjüngten Katheternabe (6) befestigtes Katheterrohr oder - kannüle (1);
- eine Nadel (3), deren Aussendurchmesser zur Anpassung an das vorgenannte Katheterrohr (1) bemessen und die an ein wesentlich zylinderförmiges Nadelgehäuse (2) befestigt ist, wobei an der Umfangsseite desselben Handgriffe (8) vorgesehen sind, die durch ein längliches Verbindungsglied (9) mit dem Mittelteil (4) des vorgenannten Nadelgehäuses (2) verbunden ist;
- eine rohrförmige Nadelschutzhülse (7), dessen Länge grösser als die Länge der Nadel (3) ist und dessen Innerdurchmesser zur Umhüllung aller Katheterteile, ausschliesslich der Handgriffe (8), ausreicht, wobei die vorgenannte Nadelschutzhülse (7) mit einem länglichen Führungsschlitz (11) versehen ist, der mit vorgenannten länglichen Verbindungsglied (9) zum Gleiten gegenüber dem vorgenannten Nadelgehäuse (2) zusammenarbeitet, wobei Klemmittel (12,10) an den vorgenannten Schlitz (11) und Verbindungsglied (9) zum Klemmen der Nadelschutzhülse (7) in dessen gegenüber des vorgenannten Nadelgehäuses (2) vollkommen vorgeschobenen Stellung, vorgesehen sind und wobei die Stirnseite (5) des vorgenannten Nadelgehäuses (2) so bemessen ist, dass sie die verjüngte Innenfläche der vorgenannten Katheternabe (6) übergreift,
dadurch gekennzeichnet, dass die vorgenannten Handgriffe (8) als ein koaxiales Aussenstück ausgebildet sind, das durch das vorgenannte längliches Verbindungsglied (9) mit dem Mittelteil (4) des vorgenannten Nadelgehäuses (2) verbunden ist.

2. Katheter nach Anspruch 1, worin der zylindrische Mittelteil (4) des vorgenannten Nadelgehäuses (2) mit zwei Führungsrippen (13) versehen ist, deren Aussendurchmesser zur Anpassung an die Innenfläche der vorgenannten Nadelschutzhülse (7) bemessen ist.

3. Katheter nach Anspruch 1, worin der Mittelteil (4) des vorgenannten Nadelgehäuses (2) mit vier länglichen Führungsrippen (14) versehen ist, wobei die Höhe dieser Rippen so bemessen ist, dass der vorgenannte zylindrische Mittelteil (4) an die Innenfläche des Nadelschutzhülse (7) anliegt.

4. Katheter nach je einem der Ansprüche 1 bis 3, worin die vorgenannten Handgriffe (8) eine teilweise unterbrochene innere Zylinderfläche und eine wesentlich parallelepipedförmige Aussenfläche mit zwei gegenüberliegenden Profilseiten aufweist.

5. Katheter nach je einem der Ansprüche 1 bis 4, worin das vorgenannte Verbindungsglied (9), das den Mittelteil (4) des vorgenannten Nadelgehäuses (2) mit dem Handgriffe (8) verbindet und mit dem vorgenannten Längstschlitz (11) zur Führung der vorgenannten Nadelschutzhülse (7) mitarbeitet, aus zwei sich zum deren Mittelteil verbreitenden und durch eine Öffnung (10) im vorgenannten Mittelteil voneinander getrennten Einzelteilen besteht und worin die vorgenannten, am vorgenannten Verbindungsglied vorgesehenen Klemmittel aus der vorgenannten Öffnung (10) im Mittelteil des vorgenannten Verbindungsgliedes (9) bestehen, während die entsprechenden Klemmittel am vorgenannten Nadelschutz aus zwei gegenüber stehenden und längst des vorgenannten Führungsschlitzes (11) vorstreckenden Zähnen (12) bestehen.

6. Katheter nach je einem der vorhergehenden Ansprüche 1 bis 5, worin der vorgenannte Längsschlitz (11) mit einem zweiten Paar von gegenüber stehenden Zähnen (15) versehen ist, die ein abgerundetes Profil aufweisen und bezüglich den vorgenannten zwei ersten gegenüber stehenden Zähnen (12) zum Stirnende der vorgenannten Nadelschutzhülse (7) angeordnet sind.

7. Katheter nach je einem der vorhergehenden Ansprüche 1 bis 6, worin ausserdem eine vom Stirnende der vorgenannten Nadelschutzhülse (7) nach oben vorstreckende Zunge (16) vorgesehen ist.

8. Katheter nach je einem der vorhergehenden Ansprüche 1 bis 7, worin der vorgenannte zylindrische Mittelteil (4) des vorgenannten Nadelgehäuses (2) aus einem durchsichtigen oder durscheinenden Werkstoff besteht.

9. Katheter nach je einem der vorhergehenden Ansprüche 1 bis 8, worin ausserdem ein feinporiger Stopfen zum Verschliessen des hinteren Endes des vorgenannten zylindrisches Mittelteiles (4) vorgesehen ist.

## Revendications

1. Cathéter intraveineux undirectionnel avec un protecteur pour l'aiguille, comprenant:
- un tube ou canule (1) du cathéter fixé à un moyeu (6) du cathéter, fuselé a ses deux surfaces intérieure et extérieure;
- une aiguille (3), dont le diamètre est choisi pour s'adapter dans ledit tube (1) du cathéter, fixée à un logement (2) de l'aiguille, sensiblement en forme d'un cylindre creux, avec des moyens périphériques de préhension (8) pour les doigts, joints au corps central (4) dudit logement (2) de l'aiguille au moyen d'un élément de connexion longitudinal (9);
- un protecteur tubulaire (7) de l'aiguille, dont la longueur est plus grande que la longueur de l'aiguille (3) et dont le diamètre interiéur est suffisant pour embracer tous les éléments du cathéter, à l'exception desdits moyens périphérique de préhension (8) pour les doigts, ledit protécteur (7) de l'aiguille étant muni d'une fente de guidage (11) longitudinale coopérante avec ledit élément de connexion longitudinal (9) pour glisser par rapport au logement (2) de l'aiguille, dans lequel des moyens de blocage (12, 10) sont prévus sur ladite fente (11) et ledit élément de connexion (9) pour bloquer le protecteur (7) de l'aiguille en sa position complètement advancée par rapport audit logement (2) de l'aiguille et dans lequel la partie frontale (5) dudit logement (2) de l'aiguille est dimensionnée de façon à engager la surface intérieure fuselé dudit moyeu (6) du cathéter,
caractérisé en ce que ledit moyen de préhension (8) pour les doigts est realisé en forme d'un élément coaxial extérieur, joint audit corps central (4) dudit logement (2) de l'aiguille au moyen dudit élément de connexion longitudinal (9).

2. Cathéter selon la revendication 1, dans lequel la partie cylindrique centrale (4) dudit logement (2) de l'aiguille est munie des deux nervures de guidage transversales (13), dont le diamètre extérieur est dimensionné de façon à s'adapter à la surface intérieure dudit protecteur tubulaire (7) de l'aiguille.

3. Cathéter selon la revendication 1, dans lequel la partie cylindrique (4) dudit logement (2) de l'aiguille est munie de quatre nervures de guidage longitudinales (14), dont l'hauteur est choisie de façon à adapter ladite portion cylindrique centrale (4) à la surface intérieure du protecteur tubulaire (7) de l'aiguille.

4. Cathéter selon une quelconque des revendications 1-3, dans lequel ledit moyen périphérique de préhension (8) pour les doigts a une surface intérieure cylindrique partiellement interrompue et une surface extérieure, sensiblement paral-lélépipédique, avec deux faces opposées profilées.

5. Cathéter selon une quelconque des revendications 1 à 3, dans lequel ledit élément de connexion longitudinal (9), qui joint le corps central (4) dudit logement (2) de l'aiguille au moyen périphérique de préhension (8) pour les doigts et qui coopére avec ladite fente longitudinale (11) pour guider ledit protecteur (7) de l'aiguille, est constitué par deux parties subséquentes séparées, fuselées de façon à s'enlarger vers la partie centrale dudit élément de connexion (9) et séparées par une ouverture (10) dans ladite partie centrale, et dans lequel lesdits moyens de blocage sur ledit élément de connexion consistent à ladite ouverture (10) dans la partie centrale dudit élément de connexion (9), tandis que les moyens de blocage correspondants sur ledit protecteur de l'aiguille consistent à deux dents opposés (12) en saillie le long de ladite fente di guidage (11).

6. Cathéter selon une quelconque des revendications 1 à 5, dans lequel ladite fente longitudinale (11) est munie d'une seconde paire de dents opposés (15) ayant un profil arrondi et situés vers le but frontal dudit protecteur (7) de l'aiguille par rapport auxdits premiers dents opposés (12).

7. Cathéter selon une quelconque des revendications 1 à 6, comprenant aussi une languette (16) s'étendant en haut du but frontal dudit protecteur tubulaire (7) de l'aiguille.

8. Cathéter selon une quelconque des revendications 1 à 7, dans lequel ladite parie cylindrique centrale (4) dudit logement (2) de l'aiguille est réalisée d'un matériel transparent ou translucide.

9. Cathéter selon une quelconque des revendications 1 à 8, comprenant aussi un bouchon microporeux fermant le but postérieur de ladite partie cylindrique centrale (4).
